# EUROPÄISCHE PATENTSCHRIFT

(11) **EP 2 368 513 B1**
(45) Veröffentlichungstag und Bekanntmachung des Hinweises auf die Patenterteilung: **13.02.2013**
(21) Anmeldenummer: 10157373.1
(22) Anmeldetag: 23.03.2010
(51) Int. Cl.: A61B 18/22, A61B 17/00, A61B 19/00, A61C 1/00

(54) **Weichgewebelaser**
Soft tissue laser
Laser des tissus mous

(43) Veröffentlichungstag der Anmeldung: 28.09.2011
(73) Patentinhaber: Ivoclar Vivadent AG, 9494 Schaan (LI)
(72) Erfinder: Goerge, Reinhard, CH-9435, Heerbrugg (CH); Rohner, Gottfried, CH-9450, Altstätten (CH)
(74) Vertreter: Baronetzky, Klaus

(56) Entgegenhaltungen:
- DE-A1- 10 009 004
- DE-A1- 19 916 162
- DE-U1- 20 304 675
- US-A1- 2006 064 080

## Beschreibung

Die Erfindung betrifft einen Weichgewebelaser, gemäß dem Oberbegriff von Anspruch 1.

Es ist seit längerem bekannt, die Spitzen von Weichgewebelasern lösbar an dem Weichgewebelaser im Übrigen zu befestigen, wobei durch die Austauschbarkeit der Spitzen sich nicht nur Probleme hinsichtlich der Sterilisation vermeiden lassen, sondern auch die Auswahl der Spitze in Anwendung an den mikrochirurgisch gewünschten Anwendungsfall möglich ist. So wird beispielsweise durch die Wahl einer entsprechenden Spitze die Lichtabgabeleistung an dem Lichtabgabeende der Spitze verändert. Ferner lassen sich aber auch besonders tiefliegende Behandlungsstellen erreichen, wenn das vordere Ende abgekröpft und spitz zulaufend ausgebildet ist.

Spitzen für faseroptische Kabel auszuwählen, ist aber seit längerem bekannt, wozu beispielhaft auf die US 3,831,017 zu verweisen ist. Auch wenn bei dieser Lösung der Sterilisierungs-Gesichtspunkt nicht im Vordergrund steht, ist doch die Austauschbarkeit der Spitze bekannt geworden, wobei bei dieser Lösung unterschiedliche Kabelaufnahmen für unterschiedliche Konfigurationen zum Einsatz gelangen.

Für die Bereitstellung der erwünschten Wirkungen ist es bei Weichgewebelasern bekannt geworden, die Strahlung mit einer Steuervorrichtung in spezieller Weise bereitzustellen, beispielsweise über gepulste Strahlung mit einer vorgegebenen Leistung und einem bestimmten Spektrum.

Ferner ist es bereits vorgeschlagen worden, die austauschbaren Spitzen mit einer Codierung zu versehen und/oder die Spitzen farblich unterschiedlich zu kennzeichnen, um die Leistung deutlich zu machen.

Nachteilig hierbei ist es, dass die Farbgebung und die entsprechende Leistung nicht ohne weiteres auf der Hand liegt, was insbesondere dann relevant ist, wenn der Weichgewebelaser nicht täglich, sondern lediglich von Fall zu Fall eingesetzt wird, so dass dann die entsprechende Leistung und die farbliche Zuordnung je nachgeschlagen werden muss.

Ein weiteres Problem besteht darin, dass der Bediener sich beim Wechseln der - farblich unterschiedlich gekennzeichneten Spitzen typischerweise darauf verlässt, dass die aus seiner Sicht durch die Farbe bedingt vorgegebene Laserleistung auch abgegeben wird, also weder zu hoch noch zu gering ist, um das Weichgewebe entsprechend dem Wunsch des Bedieners zu behandeln. Andererseits ist es aber wichtig, dass es beispielsweise nicht versehentlich zu Verbrennungen kommt.

Die Druckschrift DE 100 09 004 A1 offenbart einen Weichgewebelaser gemäß dem Oberbegriff des Anspruchs 1.

Daher liegt der Erfindung die Aufgabe zugrunde, einen Weichgewebelaser gemäß dem Oberbegriff von Anspruch 1 zu schaffen, der deutlich bedienungssicherer und auch ergonomisch besser handhabbar ist.

Diese Aufgabe wird erfindungsgemäß durch Anspruch 1 gelöst. Vorteilhafte Weiterbildungen ergeben sich durch die Unteransprüche.

Erfindungsgemäß ist es vorgesehen, die Steuerung durch den Bediener, beispielsweise am Bildschirm, so vorzusehen, dass die erwünschte Leistung vorab eingestellt wird. Der Bediener erhält dann die für die gewünschte Leistung geeignete Spitze - oder nach seiner Wahl eine Anzahl von Spitzen, die unterschiedliche Austrittsenden aufweisen, jedoch der gleichen Leistungsstufe zuzurechnen sind - und erhält damit ein bedienungssicheres Gerät, das Laserstrahlung zur Behandlung von Weichgewebe abgibt, und zwar in dem Maße und in der Form, also hinsichtlich des Impuls/Pausen-Verhältnisses des abgegebenen Wellenspektrums usw., wie er es eingestellt hat.

Überraschend lassen sich mit den erfindungsgemäßen Maßnahmen bedienungssicherere Weichgewebelaser bereitstellen, denn die Steuerung stellt erfindungsgemäß auf die dem Arbeitsende benachbart abgegebene Lichtleistung ab, also nicht beispielsweise auf die elektrische Leistung, die zugeführt wird.

Die erfindungsgemäße Leistungsanpassung kann in beliebiger geeigneter Weise realisiert sein. Beispielsweise kann die abgegebene Lichtleistung gemessen werden, nachdem die betreffende Spitze aufgesteckt ist, und die elektrische Leistung des Lasers hieran angepasst werden. Es ist auch möglich, die Spitzen je mit einer Codierung auszustatten, die die Leistungsreduktion der betreffenden Spitze anzeigt und dadurch eine verlässliche Angabe über die abgegebene Schneidleistung des Weichgewebelasers ermöglicht.

Erfindungsgemäß besonders günstig ist es, wenn die Vorgabe des Benutzers hinsichtlich der Lichtleistung als vorrangig von der Steuervorrichtung eingestellt ist und auch die aktuell benutzte Spitze berücksichtigt wird. Hierzu kann eine Spitzenerkennung verwendet werden, beispielsweise mit einer entsprechenden Codierung, die die je verwendete Spitze erfasst. Damit ist sichergestellt, dass die abzugebende Lichtleistung den Erwartungen des Benutzers entspricht.

Wenn beispielsweise die Soll-Leistung, die vom Benutzer eingestellt wird, 2 Watt beträgt und durch die gewählte Spitze die halbe Lichtleistung von dem Lichtleitelement des Lasers zur Spitze übertragen wird, etwa, weil die lichtleitende Fläche der Spitze halb so groß wie die lichtleitende Fläche des Lichtleitelements des Lasers ist, stellt die Steuervorrichtung automatisch eine elektrische Leistung, die der an der Lichtquelle abzugebenden Lichtleistung von 2 Watt entspricht. An dem vorderen Ende der Spitze wird hierdurch eine Lichtleistung von 1 Watt bereitgestellt.

Besonders günstig ist es, wenn nicht nur die unterschiedlichen Stärken des Spitzen-Lichtleitelements, sondern auch die übrigen Parameter der betreffenden Spitze, also beispielsweise die Spitzenlänge, ggf. die Abkröpfung des vorderen Endes oder beispielsweise die Länge des Lichtleitelements der Spitze, durch eine Codierung deutlich gemacht werden. Die Codierung ist bevorzugt sowohl visuell ersichtlich, beispielsweise über Farb- oder Formgebung eines Haltekörpers des Spitzenelements, aber auch bevorzugt zusätzlich hierzu über eine Codierung, die über einen Sensor erfassbar ist, so dass Informationen über die Art der Spitze der Steuervorrichtung zugeleitet werden können.

Erfindungsgemäß besonders günstig ist es, dass auf dem Display wenigstens zwei auswählbare Arbeitsverfahren, insbesondere hinsichtlich des zu behandelnden Gewebes, dargestellt sind.

Erfindungsgemäß besonders günstig ist es, dass für wenigstens ein Arbeitsverfahren ein Incontact-Modus oder ein Noncontact-Modus auswählbar ist.

Erfindungsgemäß besonders günstig ist es, dass auf dem Display die Form der Spitzen - gerade, schräg oder gebogen - auswählbar ist.

Erfindungsgemäß besonders günstig ist es, dass in Abhängigkeit von dem gewünschten Arbeitsverfahren von der Steuervorrichtung die zu verwendende Spitze auf dem Display angezeigt und die Leistung der Laserlichtquelle festgelegt wird.

Erfindungsgemäß besonders günstig ist es, dass die von der Steuervorrichtung festgelegte Leistung der Laserlichtquelle auf dem Display anzeigbar und/oder manuell veränderbar ist.

Erfindungsgemäß besonders günstig ist es, dass die verschiedenen Spitzen unterschiedliche Identifizierungscodes besitzen und dass der festgelegte Identifizierungscode auf dem Display anzeigbar ist.

Erfindungsgemäß besonders günstig ist es, dass die Einwegspitzen je einen Identifizierungscode aufweisen, und dass die Aufnahme der Spitzen eine Erfassungsvorrichtung für den Identifizierungscode aufweist, welche Erfassungsvorrichtung der Steuervorrichtung eine Information über die Art der Spitze zuleitet.

Erfindungsgemäß besonders günstig ist es, dass der Identifizierungscode durch einen Strichcode gebildet ist und dass die Erfassungsvorrichtung einen Strichcode-Lesegerät aufweist.

Erfindungsgemäß besonders günstig ist es, dass die Erfassungsvorrichtung einen Sensor aufweist, der Vorsprünge und/oder Ausnehmungen an der Spitze erkennt und dass der ldentifizierungscode durch mindestens einen Vorsprung und/oder eine Ausnehmung gebildet ist.

Erfindungsgemäß besonders günstig ist es, dass eine Durchmesser-Erfassungsvorrichtung der Spitze vorgesehen ist, über welche das Durchmesserverhältnis zwischen dem Durchmesser des Lichtleitelements und dem der Spitze erfassbar ist.

Erfindungsgemäß besonders günstig ist es, dass die Steuervorrichtung ein Mikrophon aufweist und durch Spracheingabe bedienbar ist.

Erfindungsgemäß besonders günstig ist es, dass die Steuervorrichtung einen Lautsprecher aufweist und die einstellbare Parameterkombination durch Sprachausgabe angibt.

Erfindungsgemäß besonders günstig ist es, dass der Identifizierungscode aus Farben besteht und/oder dass das zweite Lichtleitelement der Spitzen einen Durchmesser von 200 µm, 300 µm, 400 µm oder 600 µm haben.

Erfindungsgemäß besonders günstig ist es, dass das zweite Lichtleitelement 3 mm bis 30 mm über das freie Ende der Spitze herausragt.

Erfindungsgemäß besonders günstig ist es, dass die Laserlichtquelle eine Leistung von durchschnittlich 6 W bis 12 W besitzt und/oder dass die Laserlichtquelle kontinuierlich und/oder gepulstes Licht erzeugt.

Erfindungsgemäß besonders günstig ist es, dass im Übergangsbereich beider Lichtleitelemente wenigstens ein optisches Übergangselement angeordnet ist, das den Lichtstrahl des ersten Lichtleitelementes bündelt oder erweitert.

Weitere Vorteile, Einzelheiten und Merkmale ergeben sich aus der nachfolgenden Beschreibung eines Ausführungsbeispiels anhand der Zeichnungen.

Es zeigen:
- Fig. 1: eine schematische Ansicht eines Teils eines erfindungsgemäßen Weichgewebelasers in einer Ausführungsform der Erfindung, unter besonderer Darstellung der Spitze;
- Fig. 2: die Ausführungsform gemäß Fig. 1, wobei eine andere Spitze eingesetzt ist; und
- Fig. 3: die Ausführungsform gemäß Fig. 1, wobei eine dritte Spitze eingesetzt ist.

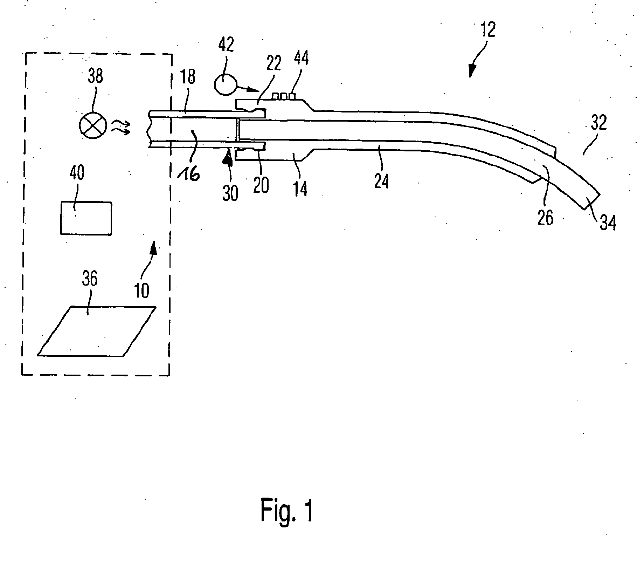

Der in Fig. 1 dargestellte Weichgewebelaser weist eine Spitze 12 auf, die über einen Verbindungskörper 14 mit dem Weichgewebelaser 10 im Übrigen verbunden ist. Der Weichgewebelaser 10 weist ein Lichtleitelement 16 auf, das in einem Führungsrohr 18 geführt und geschützt ist. Das Führungsrohr 18 weist an seinem vorderen Ende einen Wulst 20 auf, der für die formschlüssige Ankopplung an dem Verbindungskörper 14 bestimmt ist. Hierzu weist der Verbindungskörper 14 an seinem dem Führungsrohr 18 zugewandtem Ende nach innen weisende Vorsprünge 22 auf, die in Fig. 1 schematisch dargestellt sind und die federnd den Wulst 20 übergreifen können und die Spitze 12 auf dem Führungsrohr 18 sicher arretieren können.

Es versteht sich, dass eine beliebige andere Art der Ankopplung, beispielsweise auch über einem Bajonettvorschluss, ohne Weiteres möglich ist.

Die Spitze 12 weist anschließend an den Verbindungskörper 14 einen Schaft 24 auf, der ein zweites Lichtleitelement 26 umgibt und schützt. Das Lichtleitelement 26 liegt an einer Schnittstelle 30 bündig und flächig an dem vorderen Ende des Lichtleitelements 16 an, so dass ein Koppelwirkungsgrad von nahezu 100% gegeben ist. Hierzu sind die planen, einander zugewandten Oberflächen des Lichtleitelements 16 und des Lichtleitelements 26 exakt zueinander ausgerichtet, was durch die Führung des Lichtleitelements 26 im vorderen Ende des Führungsrohrs 18 unterstützt wird. Die Federwirkung der Vorsprünge 22 drückt zudem symmetrisch das freie Ende des Lichtleitelements 26 auf das freie Ende des Lichtleitelements 16.

Der Schaft 24 der Spitze 12 verläuft anschließend an den Verbindungskörper 14 zunächst koaxial mit dem Lichtleitelement 16. Bei der dargestellten Spitze 12 ist er anschließend hieran zu seinem vorderen freien Ende 32 hin abgebogen, wobei der Biegeradius etwa dem fünffachen Durchmesser des Lichtleitelements 16 entspricht.

Wie aus Fig. 1 ersichtlich ist, ragt das zweite Lichtleitelement 26 aus dem Schaft 24 frei heraus, wobei das Vorsprungmaß bei der dargestellten Spitze etwa dem dreifachen Durchmesser des Lichtleitelements 26 entspricht. Es versteht sich, dass die genannten Werte in weiten Bereichen an die Erfordernisse anpassbar sind.

Durch das Vorragen ist eine besonders gute Laserlichtbeaufschlagung auch an schwer zugänglichen und engen Stellen gewährleistet. Es versteht sich, dass das freie Ende 34 des zweiten Lichtleitelements 26 in beliebiger geeigneter Weise geschützt sein kann, beispielsweise über eine Schutzfolie, die es umhüllt.

Erfindungsgemäß ist die Spitze 12 beliebig gegen eine andere Spitze austauschbar, bei der der Durchmesser des zweiten Lichtleitelements 26 anders ist, bei der das Vorsprungmaß des Endes 34 anders ist, oder bei der die Abköpfung oder Abbiegung oder der gerade Verlauf der Spitze anders gestaltet ist. Erfindungsgemäß wird die verwendete Spitze 12 von einer Steuervorrichtung 36 festgelegt, die schematisch nach der Art eines Blockschaltbilds in Fig. 1 dargestellt ist und die im Bereich des Gehäuses des Weichgewebelasers 10 angeordnet ist.

Die Steuervorrichtung 36 legt von sich aus die geeignete Spitze fest. Die Lichtquelle 38 der Steuervorrichtung 36 wird erfindungsgemäß so gesteuert, dass die für das gewählte Arbeitsverfahren notwendige Leistung am freien Ende 34 des zweiten Lichtleitelements zur Verfügung steht. Hierzu wird bevorzugt die Spitze 12 auf einem Bildschirm 40 dargestellt und angezeigt und ggf. zusätzlich über einen Sensor 42 erkannt. Der Sensor 42 erfasst also, wie das Durchmesserverhältnis des Durchmessers des Lichtleitelements 26 zum Durchmesser des Lichtleitelements 16 ist und steuert die Lichtquelle 38 in entsprechend geeigneter Weise.

Es versteht sich, dass sowohl das verwendete Arbeitsverfahren als auch die zu verwendete Spitze in beliebiger Weise auf dem Bildschirm 40 dargestellt werden. Bei Verwendung des Sensors 42 wird bevorzugt auch gleich die eingesetzte Spitze visuell auf dem Bildschirm dargestellt, so dass hier eine bedienungssichere und ergonomische Handhabung des Weichgewebelasers möglich ist.

Es versteht sich, dass die Spitze 12, und natürlich jede verwendete Spitze eine hierfür geeignete Identifizierung, beispielsweise über einen ldentifizierungscode 44 aufweist, der auf dem Verbindungskörper 14 oder an einer beliebig anderen geeigneten Stelle der Spitze 12 angebracht ist.

In einer modifizierten, hier nicht dargestellten Ausführungsform, weist die Steuervorrichtung 36 eine Mikrophon auf und ist durch Spracheingabe bedienbar, wobei hierzu bevorzugt auch ein Lautsprecher vorgesehen ist, der die eingestellte Parameterkombination durch Sprachausgabe angibt, aber auch beispielsweise die einstellbaren Kombinationen durch Sprachausgabe anbietet.

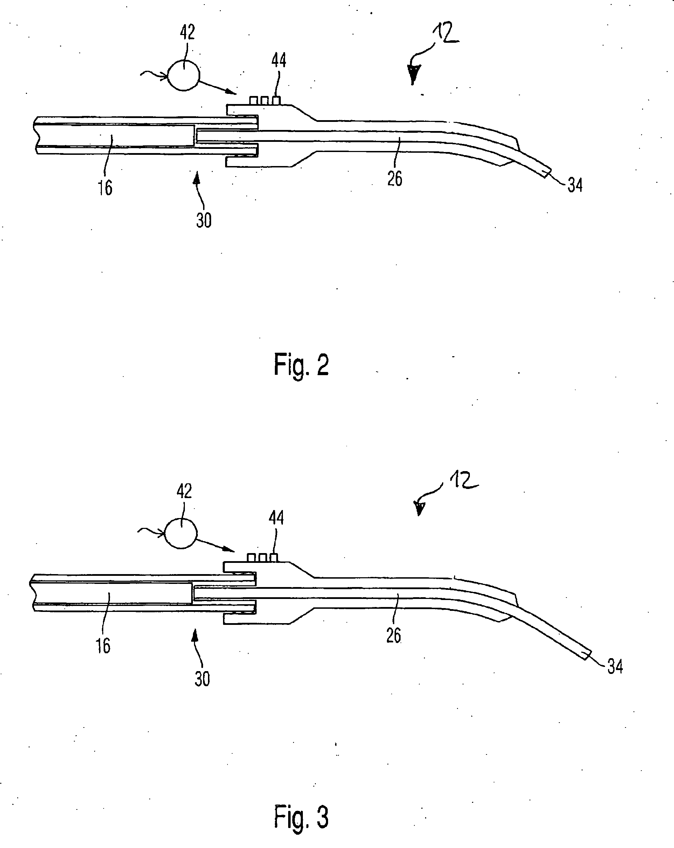

Fig. 2 zeigt eine modifizierte Spitze 12, wobei der Weichgewebelaser 10 im Übrigen dem Weichgewebelaser 10 gemäß Fig. 1 entspricht und hier nicht weiter erörtert werden muss. Bei dieser Ausgestaltung der Spitze 12 ist das Lichtleitelement 26 deutlich dünner. Sein Durchmesser beträgt etwa die Hälfte des Durchmessers des Lichtleitelements 16, so dass im Bereich der Schnittstelle 30 eine Ankopplung von lediglich 50% der freien Fläche des Lichtleitelements 16 möglich ist. Die an dem freien Ende 34 abgegebene Lichtleistung entspricht daher etwa 50% der Lichtleistung des freien Endes gemäß Fig. 1.

Dies ist durch einen entsprechend modifizierten Identifizierungscode 44 deutlich gemacht, so dass über den Sensor 42 automatisch erkannt wird, dass eine andere Spitze 12 eingesetzt ist.

Die Spitze 12 gemäß Fig. 3 unterscheidet sich von der Spitze 12 gemäß Fig. 2 durch ein länger vorragendes freies Ende 34. Diese Spitze ist im Grunde daher noch besser geeignet, an Engstellen die erwünschte Weichgewebelaserlichtleistung bereitzustellen, wobei durch das länger vorragende freie Ende 34 eine vorsichtige Handhabung wünschenswert ist.

## Patentansprüche

1. Weichgewebelaser mit einer Steuervorrichtung (36), einer steuerbaren Laserlichtquelle (38), einem ersten Lichtleitelement (16) und einer Aufnahme (20) an einem lichtaustrittseitigen Ende des ersten Lichtleitelements (16), an dem verschiedene Spitzen (12) mit einem zweiten Lichtleitelement (26) lösbar anordbar sind, die sich im Durchmesser des zweiten Lichtleitelements (26) und/oder In dem Betrag voneinander unterscheiden, um den das zweite Lichtelement (26) das freie Ende der jeweiligen Spitze (12) axial überragt, **gekennzeichnet durch** ein Display (40), wobei die Steuervorrichtung (36) eingerichtet ist, in Abhängigkeit einer gewünschten Lichtleistung an einem freien Ende (34) des zweiten Lichtelements (26), die vorzugsweise von einem gewünschten Arbeitsverfahren abhängt, eine geeignete Spitze (12) aus den anordenbaren Spitzen (12) Festzulegen und die Laserlichtquelle (38) so zu steuern, dass die gewünschte Lichtleistung am freien Ende (34) des zweiten Lichtleitelements (26) zur Verfügung steht.

2. Weichgewebelaser nach Anspruch 1, **dadurch gekennzeichnet, dass** auf dem Display wenigstens zwei auswählbare Arbeitsverfahren, insbesondere hinsichtlich des zu behandelnden Gewebes, darstellbar sind.

3. Weichgewebelaser nach Anspruch 1 oder 2, **dadurch gekennzeichnet, dass** für wenigstens ein Arbeitsverfahren ein Incontact-Modus oder ein Noncontact-Modus auswählbar ist.

4. Weichgewebelaser nach einem der vorhergehenden Ansprüche, **dadurch gekennzeichnet, dass** auf dem Display die Form der Spitzen (12) aus gerade, schräg oder gebogen auswählbar ist.

5. Weichgewebelaser nach einem der vorhergehenden Ansprüche, **dadurch gekennzeichnet, dass** in Abhängigkeit von dem gewünschten Arbeitsverfahren von der Steuervorrichtung (36) die zu verwendende Spitze (12) auf dem Display anzeigbar und die Leistung der Laserlichtquelle (38) festlegbar wird.

6. Weichgewebelaser nach einem der vorhergehenden Ansprüche, **dadurch gekennzeichnet, dass** die von der Steuervorrichtung (36) festgelegte Leistung der Laserlichtquelle (38) auf dem Display anzeigbar und/oder manuell veränderbar ist.

7. Weichgewebelaser nach einem der vorhergehenden Ansprüche, **dadurch gekennzeichnet, dass** die verschiedenen Spitzen (12) unterschiedliche Identifizierungscodes (44) besitzen und dass der festgelegte Identifizierungscode (44) auf dem Display anzeigbar ist.

8. Weichgewebelaser nach einem der vorhergehenden Ansprüche, **dadurch gekennzeichnet, dass** die Spitzen (12) je einen Identifizierungscode (44) aufweisen, und dass die Aufnahme der Spitzen (12) eine Erfassungsvorrichtung (42) für den Identifizierungscode (44) aufweist, welche Erfassungsvorrichtung der Steuervorrichtung (36) eine Information über die Art der Spitze (12) zuleitet.

9. Weichgewebelaser nach Anspruch 8, **dadurch gekennzeichnet, dass** der Identifizierungscode (44) durch einen Strichcode gebildet ist und dass die Erfassungsvorrichtung (42) einen Strichcode-Lesegerät aufweist.

10. Weichgewebelaser nach Anspruch 8, **dadurch gekennzeichnet, dass** die Erfassungsvorrichtung einen Sensor (42) aufweist, der Vorsprünge und/oder Ausnehmungen an der Spitze (12) erkennt und dass der Identifizierungscode (44) durch mindestens einen Vorsprung und/oder eine Ausnehmung gebildet ist.

11. Weichgewebelaser nach einem der Ansprüche 8 bis 10, **dadurch gekennzeichnet, dass** der Identifizierungscode (44) aus Farben besteht und/oder dass das zweite Lichtleitelement (26) der Spitzen (12) einen Durchmesser von 200 µm, 300 µm, 400 µm oder 600 µm haben.

12. Weichgewebelaser nach einem der vorhergehenden Ansprüche, **dadurch gekennzeichnet, dass** eine Durchmesser-Erfassungsvorrichtung der Spitze (12) vorgesehen ist, über welche das Durchmesserverhältnis zwischen dem Durchmesser des Lichtleitelements (16) und dem der Spitze (12) erfassbar ist.

13. Weichgewebelaser nach einem der vorhergehenden Ansprüche, **dadurch gekennzeichnet, dass** die Steuervorrichtung (36) ein Mikrophon aufweist und durch Spracheingabe bedienbar ist.

14. Weichgewebelaser nach einem der vorhergehenden Ansprüche, **dadurch gekennzeichnet, dass** die Steuervorrichtung (36) einen Lautsprecher aufweist und die einstellbare Parameterkombination durch Sprachausgabe angibt.

15. Weichgewebelaser nach einem der vorhergehenden Ansprüche, **dadurch gekennzeichnet, dass** das zweite Lichtleitelement (26) 3 mm bis 30 mm über das freie Ende der Spitze (12) herausragt.

16. Weichgewebelaser nach einem der vorhergehenden Ansprüche, **dadurch gekennzeichnet, dass** die Laserlichtquelle (38) eine Leistung von durchschnittlich 6 W bis 12 W besitzt und/oder dass mit der Laserlichtquelle (38) kontinuierliches und/oder gepulstes Licht erzeugbar ist.

17. Weichgewebelaser nach einem der vorhergehenden Ansprüche, **dadurch gekennzeichnet, dass** im Übergangsbereich beider Lichtleitelemente (16, 26) wenigstens ein optisches Übergangselement angeordnet ist, das den Lichtstrahl des ersten Lichtleitelementes bündelt oder erweitert.

## Claims

1. A soft tissue laser comprising a control device (36), a controllable laser light source (38), a first light-conducting element (16) and a receptacle (20) at a light-emitting end of the first light-conducting element (16), at which different tips (12) having a second light-conducting element (26) can be detachably arranged, which tips differ from one another in the diameter of the second light-conducting element (26) and/or in the amount by which the second light element (26) axially exceeds the free end of the corresponding tip (12), **characterized by** a display (40), wherein the control device (36), depending on a desired light output at a free end (34) of the second light element (26) which, in particular, depends on a desired work process, is configured to determine a suitable tip (12) among the arrangeable tips (12) and to control the laser light source (38) in such a fashion that the desired light output is available at the free end (34) of the second light-conducting element (26).

2. The soft tissue laser according to Claim 1, **characterized in that** on the display at least two selectable work processes can be depicted, in particular with regard to the tissue to be treated.

3. The soft tissue laser according to Claim 1 or Claim 2, **characterized in that** for at least one work process an in-contact mode or a non-contact mode can be selected.

4. The soft tissue laser according to any of the previous Claims, **characterized in that** on the display the shape of the tips (12) can be selected from straight, inclined or curved.

5. The soft tissue laser according to any of the previous Claims, **characterized in that** depending on the desired work process, the tip (12) to be used can be depicted on the display by the control device (36) and the output of the laser light source (38) can be determined.

6. The soft tissue laser according to any of the previous Claims, **characterized in that** the output of the laser light source (38) determined by the control device (36) can be depicted on the display and/or can be altered manually.

7. The soft tissue laser according to any of the previous Claims, **characterized in that** the different tips (12) have different codes of identification (44) and **in that** the code of identification (44) determined can be depicted on the display.

8. The soft tissue laser according to any of the previous Claims, **characterized in that** the tips (12) have one code of identification (44) each, and **in that** the receptacle of the tips (12) is provided with a detection device (42) for the code of identification (44), which detection device supplies information about the sort of tip (12) to the control device (36).

9. The soft tissue laser according to Claim 8, **characterized in that** the code of identification (44) is made up of a barcode and **in that** the detection device (42) is provided with a barcode reading device.

10. The soft tissue laser according to Claim 8, **characterized in that** the detection device is provided with a sensor (42) which recognises projections and/or recesses at the tip (12) and **in that** the code of identification (44) is formed by at least one projection and/or one recess.

11. The soft tissue laser according to Claims 8 to 10, **characterized in that** the code of identification (44) consists of colors and/or that the second light-conducting element (26) of the tips (12) has a diameter of 200 µm, 300 µm, 400 µm or 600 µm.

12. The soft tissue laser according to any of the previous Claims, **characterized in that** a diameter-detection device of the tip (12) is provided, via which the diameter ratio between the diameter of the light-conducting element (16) and that of the tip (12) can be detected.

13. The soft tissue laser according to any of the previous Claims, **characterized in that** the control device (36) is provided with a microphone and can be operated by means of voice input.

14. The soft tissue laser according to any of the previous Claims, **characterized in that** the control device (36) is provided with a loudspeaker and indicates the settable combination of parameters by means of voice output.

15. The soft tissue laser according to any of the previous Claims, **characterized in that** the second light-conducting element (26) exceeds the free end of the tip (12) by 3 mm to 30 mm.

16. The soft tissue laser according to any of the previous Claims, **characterized in that** the laser light source (38) has a power of 6 W to 12 W on average and/or **in that** through the laser light source (38) continuous and/or pulsed light can be produced.

17. The soft tissue laser according to any of the previous Claims, **characterized in that** in the transition area of the two light-conducting elements (16, 26) at least one optical transition element is positioned which bundles or extends the light beam of the first light-conducting element.

## Revendications

1. Laser des tissus mous comportant un dispositif de commande (36), une source de lumière laser commandable (38), un premier élément conducteur de lumière (16) et un logement (20) sur une extrémité du côté de sortie de la lumière du premier élément conducteur de lumière (16) sur lequel différentes pointes (12) peuvent être disposées de façon mobile avec un deuxième élément conducteur de lumière (26) qui se distinguent les unes des autres par le diamètre du deuxième élément conducteur de lumière (26) et/ou par l'ampleur selon laquelle le deuxième élément de lumière (26) dépasse axialement de l'extrémité libre de la pointe respective (12), **caractérisé par** un écran (40), le dispositif de commande (36) étant orienté en fonction d'une puissance lumineuse souhaitée sur une extrémité libre (34) du deuxième élément de lumière (26) qui dépend de préférence d'un processus de travail souhaité, une pointe appropriée (12) à définir parmi les pointes pouvant être ordonnées (12) et la source de lumière laser (38) à commander de telle sorte que la puissance lumineuse souhaitée soit disponible à l'extrémité libre (34) du deuxième élément conducteur de lumière (26).

2. Laser des tissus mous selon la revendication 1, **caractérisé en ce que** sur l'écran, au moins deux processus de travail sélectionnables, en particulier par rapport au tissu à traiter, peuvent être représentés.

3. Laser des tissus mous selon la revendication 1 ou 2, **caractérisé en ce que**, pour au moins un processus de travail, un mode de contact ou un mode de non contact peut être choisi.

4. Laser des tissus mous selon l'une des revendications précédentes, **caractérisé en ce que** sur l'écran, la forme des pointes (12) peut être choisie parmi les formes droites, inclinées ou courbées.

5. Laser des tissus mous selon l'une des revendications précédentes, **caractérisé en ce que**, en fonction du processus de travail souhaité, on peut afficher sur l'écran par le dispositif de commande (36) la pointe (12) à utiliser et on peut définir la puissance de la source de lumière laser (38).

6. Laser des tissus mous selon l'une des revendications précédentes, **caractérisé en ce que** la puissance déterminée par le dispositif de commande (36) de la source de lumière laser (38) peut être affichée sur l'écran et/ou peut être modifiée manuellement.

7. Laser des tissus mous selon l'une des revendications précédentes, **caractérisé en ce que** les différentes pointes (12) possèdent différents codes d'identification (44) et **en ce que** le code d'identification défini (44) peut être affiché sur l'écran.

8. Laser des tissus mous selon l'une des revendications précédentes, **caractérisé en ce que** les pointes (12) présentent respectivement un code d'identification (44) et **en ce que** le logement des pointes (12) présente un dispositif de saisie (42) pour le code d'identification (44), ledit dispositif de saisie acheminant au dispositif de commande (36) une information sur la nature de la pointe (12).

9. Laser des tissus mous selon la revendication 8, **caractérisé en ce que** le code d'identification (44) est formé d'un code barres et **en ce que** le dispositif de saisie (42) présente un appareil de lecture du code barres.

10. Laser des tissus mous selon la revendication 8, **caractérisé en ce que** le dispositif de saisie présente un capteur (42) qui reconnaît les proéminences et/ou les creux sur la pointe (12) et **en ce que** le code d'identification (44) est formé par au moins une proéminence et/ou un creux.

11. Laser des tissus mous selon l'une des revendications 8 à 10, **caractérisé en ce que** le code d'identification (44) consiste en couleurs et/ou **en ce que** le deuxième élément de lumière (26) des pointes (12) a un diamètre de 200 µm, 300 µm, 400 µm ou 600 µm.

12. Laser des tissus mous selon l'une des revendications précédentes, **caractérisé en ce qu'**un dispositif de saisie de diamètre de la pointe (12) est prévu, par lequel le rapport de diamètre entre le diamètre de l'élément conducteur de lumière (16) et celui de la pointe (12) peut être saisi.

13. Laser des tissus mous selon l'une des revendications précédentes, **caractérisé en ce que** le dispositif de commande (36) présente un microphone et peut être commandé par des entrées vocales.

14. Laser des tissus mous selon l'une des revendications précédentes, **caractérisé en ce que** le dispositif de commande (36) présente un haut-parleur et indique la combinaison de paramètres ajustables par des sorties vocales.

15. Laser des tissus mous selon l'une des revendications précédentes, **caractérisé en ce que** le deuxième élément conducteur de lumière (26) dépasse de 3 mm à 30 mm au-dessus de l'extrémité libre de la pointe (12).

16. Laser des tissus mous selon l'une des revendications précédentes, **caractérisé en ce que** la source de lumière laser (38) possède une puissance en moyenne de 6 W à 12 W et/ou **en ce que**, avec la source de lumière laser (38), une lumière continue et/ou pulsée peut être générée.

17. Laser des tissus mous selon l'une des revendications précédentes, **caractérisé en ce que** dans la zone de transition des deux éléments conducteurs de lumière (16, 26), au moins un élément de transition optique est disposé, qui resserre ou élargit le rayon lumineux du premier élément conducteur de lumière.
